# FASCICULE DE BREVET EUROPEEN

(11) **EP 4 518 991 B1**
(45) Date de publication et mention de la délivrance du brevet: **08.04.2026**
(21) Numéro de dépôt: 23723450.5
(22) Date de dépôt: 26.04.2023
(51) Int. Cl.: B01D 11/04, C07C 7/10, C07C 15/08

(54) **COLONNE D'EXTRACTION À HAUTEURS DE COMPARTIMENT ALTERNÉES**
EXTRAKTIONSKOLONNE MIT ALTERNIERENDEN FACHHÖHEN
EXTRACTION COLUMN HAVING ALTERNATING COMPARTMENT HEIGHTS

(30) Priorité: 06.05.2022 FR 2204319
(43) Date de publication de la demande: 12.03.2025
(73) Titulaire: IFP Energies nouvelles, 92500 Rueil-Malmaison (FR)
(72) Inventeur: AUGIER, Frederic, 92852 Rueil-Malmaison Cedex (FR); LORCET, Hélène, 92852 Rueil-Malmaison Cedex (FR)
(74) Mandataire: IFP Energies nouvelles
(86) Numéro de dépôt international: PCT/EP2023/060980
(87) Numéro de publication internationale: WO 2023/213649

(56) Documents cités:
- WO-A1-2014/164456
- FR-A1- 2 429 608
- US-A- 2 768 071
- US-A- 3 632 315

## Description

### Domaine technique

Le domaine de l'invention concerne l'extraction liquide-liquide dans des colonnes à plateaux perforés à déversoirs, tels qu'utilisés dans de nombreux procédés chimiques dont les procédés d'extraction des aromatiques présents dans différentes coupes pétrolières ou issues de procédés de raffinages. Les colonnes d'extraction liquide-liquide sont notamment adaptées pour la séparation liquide-liquide de composés hydrocarbonés, tels que des composés aromatiques (e.g. A6-A11) provenant de coupes hydrocarbonées étendues (e.g. coupe C6-C11, telles que provenant d'une unité de craquage catalytique (FCC Fluid Catalytic Cracking selon la terminologie anglo-saxonne).

### Technique antérieure

Le principe de fonctionnement des colonnes d'extraction liquide-liquide repose sur les différences de solubilité des composés d'une charge liquide homogène dans un solvant approprié (e.g. solvant aprotique et polaire, tel que le Sulfolane ou le DMSO). L'adjonction à la charge d'un solvant partiellement miscible provoque l'apparition d'une seconde phase vers laquelle est transférée préférentiellement une partie des composés (e.g. les composés aromatiques), constituants les plus solubles.

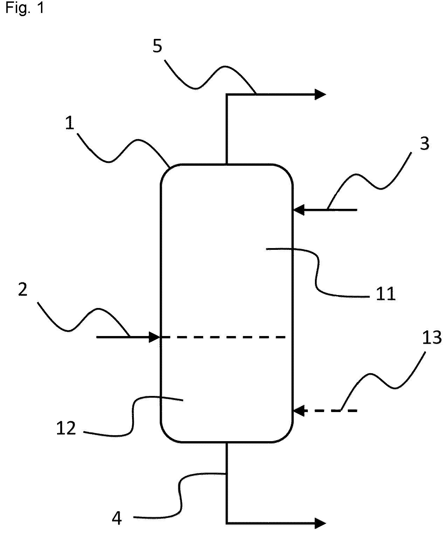

En référence à la figure 1, une colonne d'extraction liquide-liquide 1 comprend les éléments suivants :
- un premier point d'injection 2 d'une première phase (charge liquide à séparer) ;
- un deuxième point d'injection 3 d'une deuxième phase (solvant liquide de séparation), les premier et deuxième points d'injection 2 et 3 étant disposés pour permettre la circulation des première et deuxième phases à contre-courant ;
- un premier point de soutirage d'un extrait 4 (en phase liquide), tel qu'un solvant enrichi en composés extraits de la charge ; et
- un deuxième point de soutirage d'un raffinat 5 (en phase liquide), tel qu'une charge appauvrie en composés extraits, l'un des premier et deuxième points de soutirage étant disposé en fond de colonne d'extraction 1 et l'autre en tête de colonne d'extraction 1. En outre, le premier point d'injection 2 de la charge à séparer est typiquement disposé entre le deuxième point d'injection 3 du solvant liquide de séparation et le premier point de soutirage de l'extrait 4. De la même manière, le deuxième point d'injection 3 du solvant liquide de séparation est typiquement disposé entre le premier point d'injection 2 de la charge à séparer et le deuxième point de soutirage du raffinat 5. Dans cet exemple de référence, la première phase est la phase légère et la deuxième phase est la phase lourde.

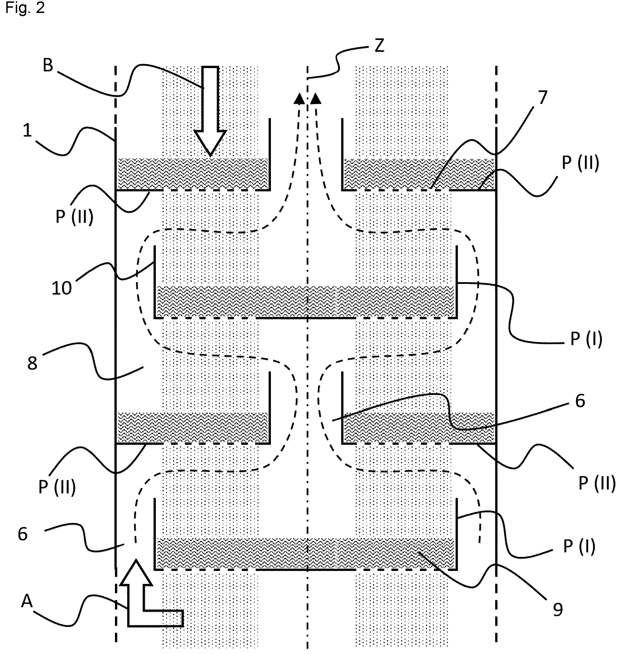

En référence à la figure 2, une colonne d'extraction liquide-liquide 1 comprend une pluralité de plateaux perforés P, chaque plateau perforé P étant équipé d'un à plusieurs déversoirs 6 en fonction des capacités visées, un déversoir 6 étant un évidement permettant le passage la première phase A dite *« continue »* au travers du plateau perforé P. Un plateau perforé P comprend au moins une zone perforée de trous 7, par où circule la deuxième phase B dite *« dispersée ».* La deuxième phase dispersée B circule sous forme de gouttes traversant la première phase continue A dans chaque espace inter-plateaux 8 définit par l'espace entre deux plateaux perforés P adjacents. Dans l'exemple de la figure 2, la deuxième phase dispersée B est la phase lourde et s'accumule au-dessus du plateau perforé P, jusqu'à former une couche coalescée 9 au-dessus de la zone perforée. Cette couche coalescée 9 peut typiquement faire de 1 cm à 8 cm d'épaisseur. Il est entendu que si la phase dispersée est la phase légère, la phase dispersée s'accumule au-dessous du plateau perforé. La deuxième phase dispersée B traverse ensuite la zone perforée du plateau perforé P pour alimenter le plateau perforé P suivant (plateau inférieur si la phase dispersée est la phase lourde), et ainsi de suite. La première phase continue A serpente dans les espaces inter-plateaux 8 en passant par les déversoirs 6, vers le haut dans cet exemple de la figure 2 car la phase continue est la phase légère.

Les brevets US 3,632,315 et US 4,588,563 décrivent des exemples de colonnes d'extraction liquide-liquide comprenant des plateaux perforés équipés d'un et/ou deux déversoirs.

Ainsi, une colonne d'extraction liquide-liquide permet de mettre en contact deux phases liquides A et B partiellement immiscibles, en contre-courant. Selon le choix de la phase dispersée (phase lourde ou phase légère), le plateau perforé P est adapté pour accueillir (au moyen de rebords 10) la couche coalescée 9 sur la partie haute du plateau perforé (phase dispersée étant la phase lourde) ou sur la partie basse (phase dispersée étant la phase légère). Dans les figures de la présente description, seul le cas du solvant liquide de séparation étant la phase dispersée et la phase lourde est décrit.

La demanderesse a identifié que le fonctionnement d'une colonne d'extraction liquide-liquide peut générer une grande variabilité de performance, en fonction notamment de :
- l'aire interfaciale entre les phases dans les espaces inter-plateaux, qui dépend de la taille des gouttes de la phase dispersée et de leur fraction volumique ; et
- l'écoulement de chaque phase dans les espaces inter-plateaux, les déversoirs et les zones perforées.

Il est connu de l'homme de métier, et parfaitement décrit dans les ouvrages d'extraction liquide-liquide (voir Handbook of Solvent Extraction, de Lo, Baird et Hanson, Krieger Publishing Company, 1991), que l'écoulement piston de chaque phase est favorable aux performances de transfert dans les colonnes d'extraction à contre-courant. L'écoulement piston est un concept classique qui considère que, sur une section donnée de la colonne, chaque phase (lourde ou légère) circule à une vitesse homogène et dans une seule direction. Il est connu également que toute présence de recirculation, tourbillon ou tout autre mouvement de liquide générant une distribution spatiale de vitesses, peut induire une dégradation de l'écoulement piston. Les dégradations d'écoulement, ou déviations du piston pur, sont regroupées sous le terme générique de « mélange axial ». On peut aussi parler de dispersion axiale. L'effet du mélange axial tend à homogénéiser les gradients de concentration présents dans une phase ou l'autre le long de la colonne, et tend à minimiser l'efficacité de l'extraction liquide-liquide.

En particulier, les recirculations de phase continue dans les déversoirs sont très néfastes. En ce qui concerne l'écoulement de la phase dispersée, il est essentiel que l'écoulement reste essentiellement descendant (ou ascendant) à travers les zones perforées. Tout entrainement de phase dispersée par la phase continue dans les déversoirs serait néfaste car tendrait à réduire l'écart de concentrations de la phase continue dans les espaces inter-plateaux successifs. Pour cette raison, l'homme du métier utilise des déversoirs suffisamment grands pour limiter au maximum l'entrainement de phase dispersée dans les déversoirs. Il est préférable que la section des déversoirs ne soit pas non plus trop excessive sous peine d'utiliser trop de surface ; pour un diamètre donné, surdimensionner la surface de déversoir peut conduire à:
- une diminution de la capacité de la colonne (et donc une augmentation de son cout à iso performance),
- une diminution de l'efficacité de l'extraction : en réduisant la surface restante disponible pour la dispersion de la phase lourde, la qualité du contact entre phases s'en trouve dégradée,
- une génération de recirculations de phase continue dans les déversoirs eux même, une réduction du caractère piston de la phase continue et ainsi une réduction de l'efficacité de l'extraction.

La section des déversoirs peut être par exemple choisie de manière à ce que la vitesse de la phase continue dans les déversoirs ne dépasse pas la vitesse terminale de chute de gouttelettes d'un diamètre plus petit que le diamètre de goutte moyen. Par exemple un diamètre de fines gouttes de 30 µm à 150 µm peut être considéré pour calculer la vitesse maximale de la phase légère dans les déversoirs.

Un autre aspect à bien considérer est l'impact de l'écoulement de phase continue dans chaque espace inter-plateaux. L'écoulement de phase continue est conditionné par le débit de phase continue circulant, et par la géométrie des espaces inter-plateaux, dont notamment la hauteur de l'espace inter-plateaux.

Si la phase continue circule trop vite dans la colonne, différents comportements néfastes peuvent apparaitre, tels que :
- des perturbations de l'écoulement de la phase dispersée, qui peut être entrainée par la phase continue vers le déversoir ;
- une déformation de la couche coalescée sur le plateau aval dans le sens de l'écoulement de la phase dispersée, cette déformation tend aussi à accumuler de la phase dispersée sous le déversoir de sortie d'espace inter-plateaux.

Ces deux comportements peuvent favoriser la présence de phase dispersée dans les déversoirs et son éventuel entrainement par la phase continue vers l'espace inter-plateaux aval dans le sens de l'écoulement de la phase continue. En effet la présence de phase dispersée dans le déversoir d'un plateau P est néfaste aux performances d'extraction car elle peut générer un rétromélange de phase dispersée, et réduire sensiblement la hauteur de couche coalescée sur le plateau P+1 (plateau aval dans le sens de l'écoulement de la phase dispersée). En outre, la réduction de la hauteur de couche coalescée peut provoquer un assèchement partiel des plateaux perforés, générant des hétérogénéités de distribution de phase dispersée dans l'espace inter-plateaux aval dans le sens de l'écoulement de la phase dispersée, voire un passage de la phase continue à travers les plateaux, phénomène qui augmente la dispersion axiale et réduit l'efficacité de la colonne.

Pour maintenir l'écoulement de la phase dispersée à un niveau de vitesses non problématique, plusieurs solutions peuvent être mises en œuvre, telles que :
- augmenter la hauteur de l'espace inter-plateaux ;
- augmenter le nombre de déversoirs sur chaque plateau.

Augmenter la hauteur des compartiments permet de réduire efficacement l'entrainement de phase lourde vers le déversoir de sortie, mais augmente naturellement la hauteur des colonnes d'extraction, ce qui a un coût et ne peut pas être poussé vers de trop grandes valeurs.

Augmenter le nombre de déversoirs est très efficace, car cela permet de diviser le flux de phase dispersée circulant en latéral localement. Des plateaux à 1 déversoir sont en général réservé à des colonnes de petites tailles (diamètre < 2 m). Des plateaux à 2 déversoirs sont très classiques et permettent de réduire efficacement la vitesse de la phase légère. Des plateaux à plus grand nombre de déversoirs sont aussi possibles.

Augmenter le nombre de déversoirs accroit la section de colonne non perforée, donc fait perdre du volume utile au contact entre les phases. Pour cette raison il n'est pas judicieux économiquement d'augmenter le nombre de déversoirs au-delà du minimum requis pour empêcher l'entrainement de phase lourde dans les déversoirs.

En référence à la figure 2, une mise en œuvre classique de colonnes d'extraction liquide-liquide comprenant des plateaux à 2 déversoirs, consiste à utiliser une alternance de 2 plateaux distincts, de manière à ce que l'écoulement de la phase continue circule en serpentant dans la colonne et en traversant les zones de présence de gouttes de phase dispersée. Ceci permet d'augmenter le transfert de matière entre les phases. Un premier type de plateau (type I) utilise 2 déversoirs périphériques ou latéraux (orthogonaux à l'axe central/vertical Z de la colonne), sensiblement collés à la virole de la colonne et disposés en des positions diamétralement opposées. Le deuxième type de plateau (type II) utilise 1 seul déversoir central, sous forme d'une bande (perpendiculaire à l'axe central/vertical Z de la colonne) couvrant sensiblement la totalité du diamètre de la colonne. Une colonne d'extraction liquide-liquide telle que représentée dans la figure 2 est une colonne dite à 2 passes, *i.e.,* une colonne dans laquelle la phase continue circule en serpentant selon 2 parcours différents tels que représentés par les flèches en pointillés de la figure 2.

Dans la présente description, lorsque la phase dispersée est la phase lourde, l'espace inter-plateaux d'un plateau de type I est l'espace inter-plateaux situé directement au-dessus du plateau de type I ; l'espace inter-plateaux d'un plateau de type II est l'espace inter-plateaux situé directement au-dessus du plateau de type II. Ainsi, l'espace inter-plateaux correspond à l'espace disposé du côté du rebord 10 et de la couche coalescée 9 du plateau Pᵢ, *i.e.,* l'espace inter-plateaux d'un plateau Pᵢ correspond à l'espace disposé en aval du plateau Pᵢ, dans le sens de l'écoulement de la phase continue.

L'état de l'art consiste à utiliser des plateaux alternés de type I et II, avec une hauteur d'espace inter-plateaux commune et des sections de déversoirs identiques, le déversoir central des plateaux de type Il ayant alors une section égale à la somme des 2 déversoirs des plateaux de type I.

La présente invention a pour objet de remédier aux déficiences de performance mentionnées ci-dessus et d'augmenter la performance des colonnes d'extraction liquide-liquide.

### Résumé de l'invention

Dans le contexte précédemment décrit, un premier objet de la présente description est de proposer une colonne d'extraction liquide-liquide permettant :
- de conserver une gamme comprise entre 5% et 20% de la fraction volumique moyenne de phase dispersée (e.g. solvant/phase lourde) dans un compartiment (*i.e.,* zone comprenant un plateau perforé et un espace inter-plateaux adjacent) ;
- le non-entrainement des gouttelettes de phase dispersée dans les déversoirs par la phase continue (e.g. charge/phase légère) afin de limiter le mélange axial de la phase dispersée ;
- une hauteur de couche coalescée de la phase dispersée sur chaque plateau suffisante (e.g. au moins 2 cm, tel que de 2 cm à 4 cm) pour empêcher le passage de la phase continue à travers le plateau perforé (et forcer le passage exclusif de la phase continue dans les déversoirs) ;
- une vitesse transverse de phase continue adaptée, qui ne perturbe pas l'écoulement de la phase dispersée.

De façon surprenante, la demanderesse a identifié que des caractéristiques particulières de plateaux perforés, telles que la hauteur des espaces inter-plateaux et optionnellement la section des déversoirs, permettent de contrôler l'hydrodynamique tout le long de la colonne en limitant le mélange axial. Cette solution technique permet de conserver une efficacité de transfert de matière satisfaisante sur chaque plateau.

Selon un premier aspect, les objets précités, ainsi que d'autres avantages, sont obtenus par une colonne d'extraction liquide-liquide, comprenant les éléments suivants :
- un premier point d'injection d'une première phase ;
- un deuxième point d'injection d'une deuxième phase, les premier et deuxième points d'injection étant disposés sur la colonne d'extraction pour permettre la circulation de la première phase et de deuxième phase à contre-courant dans la colonne d'extraction, l'une des première et deuxième phases étant une phase continue et l'autre étant une phase dispersée ;
- un premier point de soutirage d'un extrait et un deuxième point de soutirage d'un raffinat, l'un étant disposé en fond de colonne d'extraction et l'autre étant disposé en tête de colonne d'extraction ; et
- une pluralité de plateaux perforés disposés du haut de la colonne d'extraction au bas de la colonne d'extraction, les plateaux perforés étant espacés d'un espace inter-plateaux et comprenant des rebords adaptés pour recevoir une couche coalescée de la phase dispersée au-dessus ou au-dessous des plateaux perforés ;
- une pluralité de déversoirs, un déversoir étant un évidement adjacent à un rebord et permettant le passage la phase continue au travers du plateau perforé,

la colonne d'extraction comprenant alternativement :
   - des plateaux perforés dits de type I comprenant deux déversoirs périphériques ; et
   - des plateaux perforés dits de type Il comprenant un seul déversoir central,
colonne d'extraction dans laquelle :
   - la hauteur H1 des espaces inter-plateaux disposés directement en aval des plateaux de type I, dans le sens de l'écoulement de la phase continue, est supérieure à la hauteur H2 des espaces inter-plateaux disposés directement en aval des plateaux de type II.

Selon un ou plusieurs modes de réalisation, le ratio H1/H2 entre la hauteur H1 et la hauteur H2 est compris entre 1,1 et 2.

Selon un ou plusieurs modes de réalisation, le ratio H1/H2 entre la hauteur H1 et la hauteur H2 est compris entre 1,1 et 1,5.

Selon un ou plusieurs modes de réalisation, la hauteur H1 des espaces inter-plateaux des plateaux perforés de type I est comprise entre 0,22 m et 1,50 m, préférablement entre 0,27 m et 0,90 m, très préférablement entre 0,33 m et 0,82 m.

Selon un ou plusieurs modes de réalisation, la hauteur H2 des espaces inter-plateaux des plateaux perforés de type Il est comprise entre 0,20 m et 1,00 m, préférablement entre 0,25 m et 0,60 m, très préférablement entre 0,30 m et 0,55 m.

Selon un ou plusieurs modes de réalisation, la colonne d'extraction liquide-liquide comprend :
- un secteur d'extraction, s'étendant sensiblement du premier point d'injection de la première phase jusqu'à sensiblement le deuxième point d'injection de la deuxième phase, et
- un secteur de contre-lavage, adjacent au secteur d'extraction et s'étendant du premier point d'injection de la première phase jusqu'à sensiblement un troisième point d'injection de liquide de contre-lavage.

Selon un ou plusieurs modes de réalisation, la section S2 des déversoirs centraux est supérieure à la section S1 des déversoirs périphériques, la section S1 correspondant à la somme des sections des deux déversoirs périphériques.

Selon un ou plusieurs modes de réalisation, le ratio S2/S1 entre la section S2 et la section S1 est compris entre 1,1 et 2.

Selon un ou plusieurs modes de réalisation, le ratio S2/S1 entre la section S2 et la section S1 est compris entre 1,1 et 1,5.

Selon un deuxième aspect, les objets précités, ainsi que d'autres avantages, sont obtenus par un procédé d'extraction liquide-liquide comprenant les étapes suivantes :
- injecter une première phase et une deuxième phase dans une colonne d'extraction liquide-liquide selon le premier aspect ; et
- soutirer un extrait et un raffinat de la colonne d'extraction liquide-liquide.

Selon un ou plusieurs modes de réalisation, la colonne d'extraction est opérée à une pression comprise entre 0,05 MPa et 3 MPa, de préférence entre 0,1 MPa et 2 MPa, de préférence entre 0,2 MPa et 1,5 MPa, de préférence entre 0,3 MPa et 1 MPa ; et à une température comprise entre 10°C et 150°C, de préférence entre 15°C et 130°C, de préférence entre 30°C et 120°C, de préférence entre 40°C et 110°C.

Selon un ou plusieurs modes de réalisation, la première phase comprend un mélange de composés aromatiques et non-aromatiques.

Selon un ou plusieurs modes de réalisation, la deuxième phase comprend un composé choisi parmi l'éthylène glycol, le diéthylène glycol, le triéthylène glycol, hexaméthylphosphoramide, le carbonate de propylène, le carbonate d'éthylène, le sulfolane, le 3-methylsulfolane, le N-méthylacétamide, le N,N-diméthylacétamide, la butyrolactone, la 1-méthylpyrrolidone, le diméthylsulfoxyde, la caprolactame, le N-méthylformamide, la pyrrolidine-2-one, le furfural, le 1,1,3,3-tétraméthylurée et un mélange de ceux-ci.

Selon un ou plusieurs modes de réalisation, la deuxième phase comprend du sulfolane et de l'eau.

Des modes de réalisation de la colonne et du procédé d'extraction liquide-liquide selon les aspects précités ainsi que d'autres caractéristiques et avantages vont apparaître à la lecture de la description qui va suivre, donnée à titre uniquement illustratif et non limitatif, et en référence aux dessins suivants.

### Liste des figures

La figure 1 montre schématiquement une vue en coupe d'une colonne d'extraction liquide-liquide.
La figure 2 montre schématiquement une vue en coupe de l'écoulement de la phase dispersée et de la phase continue dans la colonne d'extraction liquide-liquide de référence.
La figure 3 montre schématiquement une vue en coupe de l'écoulement de la phase dispersée et de la phase continue dans une colonne d'extraction liquide-liquide selon la présente invention.
La figure 4 montre schématiquement une vue de dessus d'un plateau perforé de type I et d'un plateau perforé de type II selon la présente invention.

### Description des modes de réalisation

Des modes de réalisation de l'invention vont maintenant être décrits en détail. Dans la description détaillée suivante, de nombreux détails spécifiques sont exposés afin de fournir une compréhension plus approfondie de l'invention. Cependant, il apparaîtra à l'homme du métier que l'invention peut être mise en oeuvre sans ces détails spécifiques. Dans d'autres cas, des caractéristiques bien connues n'ont pas été décrites en détail pour éviter de compliquer inutilement la description.

Dans la présente description, le terme « comprendre » est synonyme de (signifie la même chose que) « inclure » et « contenir », et est inclusif ou ouvert et n'exclut pas d'autres éléments non récités. Il est entendu que le terme « comprendre » inclut le terme exclusif et fermé « consister ». En outre, dans la présente description, le terme « sensiblement » correspondent à une approximation de ± 10%, préférablement de ± 5%, très préférablement de ± 2%, d'une valeur de référence telle qu'une distance, une vitesse, un débit, une teneur en composés, une température, une pression, etc.

### Colonne d'extraction liquide-liquide

Une colonne d'extraction liquide-liquide selon la présente invention comprend tous les éléments tels que définis dans la figure 1. En outre, afin d'augmenter le rendement et la pureté, deux zones de fonctionnement distinctes peuvent être définies dans une colonne d'extraction liquide-liquide selon la présente invention en regard du premier point d'injection 2 du liquide à séparer :
- un secteur d'extraction 11, s'étendant sensiblement du premier point d'injection 2 de la première phase jusqu'à sensiblement le deuxième point d'injection 3 de la deuxième phase, permet notamment d'extraire des composés du liquide à séparer (e.g. aromatiques) par contactage avec le solvant liquide de séparation à contre-courant (zone dite de rendement), et
- un secteur optionnel de contre-lavage 12 (ou backwash selon la terminologie en anglais), adjacent au secteur d'extraction 11 et s'étendant du premier point d'injection 2 de la première phase jusqu'à sensiblement un troisième point d'injection 13 de liquide de contre-lavage (optionnel), tel qu'un recycle d'extrait, permet notamment de contre-extraire des composés non-désirés (e.g. non aromatiques lourds) contenus dans l'extrait 4 par le liquide de contre-lavage afin de garantir un haut niveau de pureté. Selon un ou plusieurs modes de réalisation, le troisième point d'injection 13 de liquide de contre-lavage est disposé entre le premier point d'injection 2 et le premier point de soutirage de l'extrait 4.

Concrètement en référence à la figure 1, le liquide de séparation sort de la colonne d'extraction liquide-liquide 1 en entrainant des composés d'intérêt à séparer (e.g. aromatiques) pour former l'extrait 4. L'extrait peut contenir également des composés non-désirés (e.g. non-aromatiques légers, tels que des C6-C7) qui peuvent être séparés en aval (e.g. par distillation et/ou strippage). Avantageusement, l'extrait 4 ne contient sensiblement pas (ou très peu) de composés non-désirés difficiles à séparer (e.g. non-aromatiques plus lourds, tels que des C8+), qui sont séparés de l'extrait dans le secteur de contre-lavage 12. En référence à la figure 1, le liquide de séparation est plus lourd que le liquide à séparer et est injecté en tête de colonne d'extraction liquide-liquide 1, alors que le liquide de contre-lavage optionnel est injecté en pied de colonne d'extraction liquide-liquide 1. Il est entendu que la présente invention concerne également les colonnes d'extraction liquide-liquide, dans lesquelles le liquide de séparation est plus léger que le liquide à séparer, le point d'injection 3 du liquide de séparation étant alors en pied de colonne d'extraction liquide-liquide 1 et le point d'injection 13 du liquide de contre-lavage étant en tête de colonne d'extraction liquide-liquide 1.

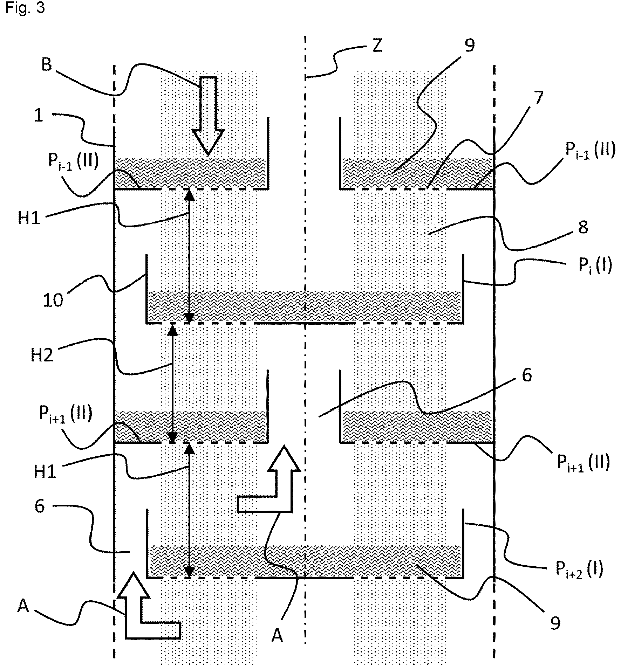

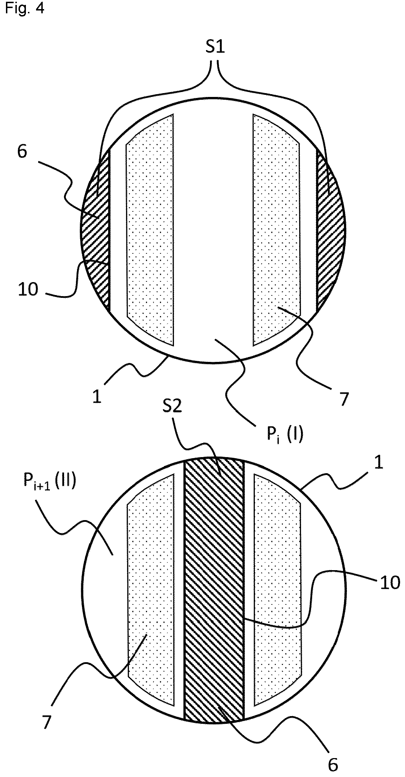

En référence aux figures 3 et 4, une colonne d'extraction liquide-liquide 1 selon la présente l'invention comprend n plateaux perforés Pᵢ, i étant compris entre 1 et n. Chaque plateau perforé Pᵢ est disposé de sorte que la phase dispersée B (*i.e.,* le liquide de séparation plus lourd que le liquide à séparer) s'écoule au travers des trous 7 du plateau perforé Pᵢ, les gouttelettes de la phase dispersée B recoalesçant sur le plateau perforé Pᵢ₊₁ suivant pour former un volume liquide empêchant le passage de la phase continue A (*i.e.,* le liquide à séparer plus léger que le liquide de séparation) à travers les trous du plateau perforé Pᵢ₊₁. Le liquide à séparer circule à contre-courant du liquide de séparation, *i.e.,* de bas en haut par les déversoirs centraux et périphériques 6, et de façon transverse dans un espace inter-plateaux 8. En référence à la figure 3, la phase lourde est la phase dispersée B et la phase légère est la phase continue A. Il est entendu qu'une colonne d'extraction liquide-liquide 1 peut comprendre des plateaux perforés adaptés pour que la phase dispersée soit la phase légère et la phase continue soit la phase lourde.

Selon un ou plusieurs modes de réalisation, le nombre n de plateaux perforés Pᵢ est compris entre 10 et 200, préférablement entre 50 et 150.

Selon l'invention, la colonne d'extraction liquide-liquide 1 comprend alternativement des plateaux perforés Pᵢ de type II, *i.e.,* comprenant un seul déversoir central, et des plateaux perforés Pᵢ de type I, *i.e.,* comprenant deux déversoirs périphériques.

Spécifiquement, les plateaux perforés de type II ont une zone perforée de trous 7 divisée en 2 parties, de part et d'autre du déversoir central 6, comme présenté sur le plateau Pᵢ de la figure 4. En outre, les plateaux perforés de type I ont une zone perforée de trous 7 pouvant être unique ou être divisée en 2 parties, comme présenté sur le plateau Pᵢ₊₁ de la figure 4, pour rester similaires aux parties de la zone perforée de trous 7 des plateaux perforés de type II.

Ainsi, il est entendu que la présente invention concerne les colonnes d'extraction liquide-liquide à 2 passes. Spécifiquement, en référence aux figures 3 et 4, la colonne d'extraction liquide-liquide 1 comprend des plateaux Pᵢ et Pᵢ₊₂ à 2 déversoirs périphériques 6 (plateaux de type I) disposés de façon alternée avec des plateaux Pᵢ₋₁ et Pᵢ₊₁ à 1 déversoir central 6 (plateaux de type II). Préférablement, les déversoirs périphériques 6 sont sensiblement adjacent à la virole de la colonne, voire sensiblement collés à ladite virole. Préférablement, les déversoirs périphériques 6 sont sensiblement disposés en des positions diamétralement opposées. Préférablement, les déversoirs centraux 6 sont disposés sous forme d'une bande couvrant sensiblement la totalité du diamètre de la colonne.

La demanderesse a identifié, notamment lors d'une étude numérique de l'écoulement dans les espaces inter-plateaux alternatifs des plateaux perforés Pᵢ de type I et II, qu'un entrainement de phase dispersée dans les déversoirs était possible et pouvait minimiser les performances d'extraction. Il a été observé que cet entrainement apparaissait en premier dans les déversoirs des plateaux de type II (déversoirs centraux).

Selon l'invention, les plateaux perforés sont modifiés de manière différenciée selon leur type. Alors que l'état de l'art proposerait d'augmenter la hauteur des espaces inter-plateaux et la section (surface) de déversoirs de tous les plateaux, la demanderesse a identifié que l'entrainement de phase dispersée dans la phase continue pouvait être diminuée en :
- augmentant la hauteur H1 des espaces inter-plateaux 8 des plateaux perforés Pi de type I à un niveau supérieur à la hauteur H2 des espaces inter-plateaux 8 des plateaux perforés Pi de type II, tel que montré dans la figure 3 ; et
- augmentant optionnellement la section S2 des déversoirs centraux 6 des plateaux perforés Pi de type II à un niveau supérieur à la section S1 des déversoirs périphériques 6 des plateaux perforés Pi de type I, tel que montré dans la figure 4.

Il est entendu dans la présente description que la hauteur d'un espace inter-plateaux 8 d'un plateau perforé Pᵢ correspond à la distance entre ledit plateau perforé Pᵢ et le plateau perforé disposé directement en aval dans le sens de l'écoulement de la phase continue, *i.e.,* le plateau perforé Pᵢ₊₁ dans l'exemple de la figure 3.

Il est entendu dans la présente description que la section S1 des déversoirs périphériques 6 d'un plateau perforé correspond à la somme des sections des deux déversoirs périphériques dudit plateau perforé.

Avantageusement, en alternant des plateaux perforés de type I et Il dans la colonne d'extraction 1, la présente invention permet d'éviter l'entrainement de phase dispersée dans les déversoirs des plateaux de type II (déversoirs centraux), tout en minimisant les inconvénients des modifications appliquées. Ainsi, l'augmentation de la hauteur de colonne est réduite par 2 par rapport à l'état de l'art ou les hauteurs de tous les espaces inter-plateaux sont augmentées.

De même l'augmentation de la section des déversoirs des plateaux perforés de type II peut s'accompagner d'une diminution de surface perforée et de la qualité du transfert dans le l'espace inter-plateaux des plateaux perforés de type I, mais cette diminution ne concerne pas les espaces inter-plateaux des plateaux perforés de type II. Donc l'impact de l'augmentation de la section des déversoirs des plateaux perforés de type Il dégrade moins les performances de transfert qu'un changement de section concernant la totalité des déversoirs, selon l'état de l'art.

Selon un ou plusieurs modes de réalisation, le ratio H1/H2 entre la hauteur H1 et la hauteur H2 est compris entre 1,1 et 2, préférablement entre 1,1 et 1,5. Selon un ou plusieurs modes de réalisation, le ratio H1/H2 entre la hauteur H1 et la hauteur H2 est compris entre 1,2 et 1,9, préférablement entre 1,3 et 1,7.

Selon un ou plusieurs modes de réalisation, la hauteur H1 des espaces inter-plateaux 8 des plateaux perforés de type I est comprise entre 0,22 m et 1,50 m, préférablement entre 0,27 m et 0,90 m, très préférablement entre 0,33 m et 0,83 m.

Selon un ou plusieurs modes de réalisation, la hauteur H2 des espaces inter-plateaux 8 des plateaux perforés de type Il est comprise entre 0,20 m et 1,00 m, préférablement entre 0,25 m et 0,60 m, très préférablement entre 0,30 m et 0,55 m.

Selon un ou plusieurs modes de réalisation, le ratio S2/S1 entre la section S2 et la section S1 est compris entre 1,1 et 2, préférablement entre 1,1 et 1,5. Selon un ou plusieurs modes de réalisation, le ratio S2/S1 entre la section S2 et la section S1 est compris entre 1,2 et 1,4.

Selon un ou plusieurs modes de réalisation, la section des déversoirs 6 est prédéterminée pour que la vitesse de la phase continue traversant lesdits déversoirs soit comprise entre 0,005 m/s et 0,050 m/s. Selon un ou plusieurs modes de réalisation, le débit de phase continue est comprise entre 70 m³/hr et 400 m³/hr, préférablement entre 80 m³/hr et 350 m³/hr.

Selon un ou plusieurs modes de réalisation, les sections S1 et S2 des déversoirs 6 sont comprises entre 0,3 m² à 15 m², préférablement entre 2 m² à 10 m². Selon un ou plusieurs modes de réalisation, la section S1 du déversoir 6 central est comprise entre 0,33 m² à 15 m², préférablement entre 2,2 m² à 10 m². Selon un ou plusieurs modes de réalisation, la section S2 des déversoirs 6 périphériques est comprise entre 0,3 m² à 13,5 m², préférablement entre 2 m² à 9 m².

Le diamètre de la colonne d'extraction 1 est typiquement fonction de la valeur prédéterminée du débit de la phase dispersée la traversant. Selon un ou plusieurs modes de réalisation, le débit de la phase dispersée est compris entre 140 m³/hr et 2300 m³/hr, préférablement entre 200 m³/hr et 2000 m³/hr.

La hauteur de la colonne d'extraction 1 est typiquement fonction de son diamètre. Selon un ou plusieurs modes de réalisation, la hauteur de la colonne d'extraction est comprise entre 15 m et 90 m.

Selon un ou plusieurs modes de réalisation, les plateaux perforé Pᵢ présentent des trous 7 de diamètre compris entre 2 mm et 10 mm. Selon un ou plusieurs modes de réalisation, le diamètre des trous est prédéterminé pour que la vitesse au trou soit comprise entre 0,05 m/s et 0,60 m/s.

### Procédé d'extraction liquide-liquide

Selon un ou plusieurs modes de réalisation, la colonne d'extraction 1 est opérée à une pression comprise entre 0,05 MPa et 3 MPa (0,5 et 30 bara), de préférence entre 0,1 MPa et 2 MPa (1 et 20 bara), de préférence entre 0,2 MPa et 1,5 MPa (2 et 15 bara), de préférence entre 0,3 MPa et 1 MPa (3 et 10 bara). Selon un ou plusieurs modes de réalisation, l'extracteur est opéré à une température comprise entre 10°C et 150°C, de préférence entre 15°C et 130°C, de préférence entre 30°C et 120°C, de préférence entre 40°C et 110°C.

### Charge à liquide à séparer

Le procédé d'extraction liquide-liquide selon l'invention permet de traiter une charge liquide à séparer comprenant un mélange de composés aromatiques et non-aromatiques. Préférablement la charge est hydrotraitée et/ou hydrogénée. Selon un ou plusieurs modes de réalisation, la charge est une charge essence optionnellement hydrotraitée et/ou hydrogénée.

Selon un ou plusieurs modes de réalisation, la charge est une coupe C5+, *i.e.,* contenant des composés à 5 atomes de carbone et plus. Selon un ou plusieurs modes de réalisation, la charge est une coupe C5-C10 ou C5-C11, *i.e.,* contenant des composés comprenant de 5 à 10 ou de 5 à 11 atomes de carbone. Selon un ou plusieurs modes de réalisation, la charge est une coupe C6-C10 ou C6-C11, *i.e.,* contenant des composés comprenant de 6 à 10 ou de 6 à 11 atomes de carbone.

Selon un ou plusieurs modes de réalisation, la charge comprend au moins 20% poids, préférablement au moins 30% poids, très préférablement au moins 40% poids, (e.g. au moins 50% poids) de composés aromatiques de 6 à 11 atomes de carbone, par rapport au poids total de la charge.

Selon un ou plusieurs modes de réalisation, la charge comprend au moins 20% poids, préférablement au moins 30% poids, très préférablement au moins 40% poids, (e.g. au moins 50% poids) de composés mono-aromatiques de 6 à 11 atomes de carbone, par rapport au poids total de la charge.

Selon un ou plusieurs modes de réalisation, les composés aromatiques de la charge sont des composés mono-aromatiques à au moins 95% poids, préférablement à au moins 98% poids, très préférablement à au moins 99% poids.

Selon un mode de réalisation de l'invention, la charge comprend moins de 50 ppm poids de soufre, préférablement moins de 10 ppm poids de soufre, et très préférablement moins de 1 ppm poids de soufre.

Selon un mode de réalisation de l'invention, la charge comprend moins de 100 ppm poids d'azote, préférablement moins de 10 ppm poids d'azote, et très préférablement moins de 1 ppm poids d'azote.

Selon un mode de réalisation de l'invention, la charge comprend moins de 0,1 % poids de dioléfines, préférablement moins de 0,05% poids de dioléfines, et très préférablement moins de 0,01% poids de dioléfines.

Selon un mode de réalisation de l'invention, la charge comprend moins de 0,1 % poids d'oléfines, préférablement moins de 0,05% poids d'oléfines, et très préférablement moins de 0,01 % poids d'oléfines.

Selon un mode de réalisation de l'invention, la charge présente une teneur inférieure ou égale à 5000 ppm poids, préférablement inférieure ou égale à 4500 ppm poids, et très préférablement inférieure ou égale à 3000 ppm poids, en composés ayant une température d'ébullition supérieure à 217°C, tel que le naphtalène.

Selon un mode de réalisation de l'invention, la charge est dépourvue des composés suivants : H₂, H₂S, gaz léger tel que l'éthane, le propane et le butane. Selon un mode de réalisation de l'invention, l'élimination de ces composés dans la charge est réalisée dans une colonne de fractionnement.

Selon un mode de réalisation, ladite charge est au moins en partie une coupe essence issue d'une unité de craquage catalytique en lit fluidisé (unité FCC pour « Fluid Catalytic Cracking » selon la terminologie anglo-saxonne), la coupe essence ayant préférablement été hydrogénée sélectivement pour transformer des dioléfines en oléfines, puis fractionnée pour obtenir une coupe C5-C10, C5-C11, C6-C10 ou C6-C11, puis hydrogénée pour saturer les composés oléfiniques. Selon un mode de réalisation de l'invention, la charge est issue de l'hydrogénation d'une essence de pyrolyse (PyGas selon la terminologie anglo-saxonne) en mélange avec une coupe essence issue d'une unité FCC.

### Solvant liquide de séparation

Selon un ou plusieurs modes de réalisation, le solvant comprend un composé choisi parmi l'éthylène glycol, le diéthylène glycol, le triéthylène glycol, hexaméthylphosphoramide, le carbonate de propylène, le carbonate d'éthylène, le sulfolane, le 3-methylsulfolane, le N-méthylacétamide, le N,N-diméthylacétamide, la butyrolactone, la 1-méthylpyrrolidone, le diméthylsulfoxyde, la caprolactame, le N-méthylformamide, la pyrrolidine-2-one, le furfural, le 1,1,3,3-tétraméthylurée et un mélange de ceux-ci.

Selon un ou plusieurs modes de réalisation, le solvant comprend ou consiste en du sulfolane. Selon un ou plusieurs modes de réalisation, le solvant est constitué d'au moins 80% poids (e.g. d'au moins 90% poids), préférablement d'au moins 95% poids (e.g. d'au moins 99% poids), en sulfolane, par rapport au poids total du solvant.

Selon un ou plusieurs modes de réalisation, le solvant comprend en outre un anti-solvant, tel que de l'eau. Selon un ou plusieurs modes de réalisation, l'anti-solvant comprend ou consiste en de l'eau. Selon un ou plusieurs modes de réalisation, le solvant comprend entre 0,01 % poids et 5% poids, préférablement entre 0,1 % poids et 3% poids (e.g. entre 0,5 % poids et 2% poids) d'anti-solvant, tel que de l'eau, par rapport au poids total du solvant. Selon un ou plusieurs modes de réalisation, le solvant comprend, ou consiste en du sulfolane et de l'eau.

### Exemples

Les exemples de colonne d'extraction liquide-liquide décrits ci-dessous présentent tous les caractéristiques suivantes : La colonne d'extraction liquide-liquide présente un diamètre de 4,8 m. Chaque plateau comprend 17192 trous. La charge (phase continue et légère) est injectée au plateau inférieur avec un débit de 1,02E+05 kg/hr. La charge comprend 40% poids d'isooctane, 30% poids de benzène et 30% poids de paraxylène (densité de 724 kg/m³). Le solvant liquide de séparation (phase dispersée et lourde) est injecté au plateau supérieur avec un débit de 8,25E+05 kg/hr. Le solvant liquide de séparation comprend 99,5% poids de sulfolane et 0,5% poids d'eau (densité de 1136 kg/m³).

### Exemple 1 (référence) : hauteur des espaces inter-plateaux et sections de déversoir de constants.

La colonne d'extraction liquide-liquide présente une hauteur de 36,0 m et est composée d'une succession de 120 plateaux perforés.

Aucun ajustement n'est mis en oeuvre :
- hauteurs des espaces inter-plateaux : H1 = H2 = 0,30 m,
- sections des déversoirs : S1 = S2 = 1,70 m².

Dans l'exemple 1 de référence, des perturbations de l'écoulement de la phase dispersée sont observées, de la phase dispersée est entrainée avec de la phase continue vers le déversoir, et une déformation de la couche coalescée est observée sur le plateau aval dans le sens de l'écoulement de la phase dispersée. Pour exprimer quantitativement la performance, on calcule la Hauteur Equivalente de Plateau Théorique (HEPT), un concept classique en séparation (distillation, extraction). Plus la HEPT est basse et plus l'extracteur est efficace.

Pour l'exemple 1 de référence, la HEPT est de 4,76 m.

### Exemple 2 (référence) : augmentation de la hauteur des espaces inter-plateaux

Par rapport à l'exemple 1, les hauteurs H1 et H2 des espaces inter-plateaux sont augmentées :
- hauteurs des espaces inter-plateaux : H1 = H2 = 0,45 m,
- sections des déversoirs : S1 = S2 = 1,70 m².

Pour l'exemple 2 de référence, la HEPT est de 5,83 m.

La colonne d'extraction liquide-liquide présente une hauteur de 44,0 m et est composée d'une succession de 98 plateaux perforés.

### Exemple 3 (référence) : augmentation des sections des déversoirs

Par rapport à l'exemple 1, les sections S1 et S2 des déversoirs sont augmentées :
- hauteurs des espaces inter-plateaux : H1 = H2 = 0,30 m,
- sections des déversoirs : S1 = S2 = 2,10 m².

Pour l'exemple 3 de référence, la HEPT est de 4,81 m.

La colonne d'extraction liquide-liquide présente une hauteur de 36,3 m et est composée d'une succession de 121 plateaux perforés.

### Exemple 4 (invention) : hauteurs alternées des espaces inter-plateaux

Par rapport à l'exemple 1, seules les hauteurs H1 sont augmentées :
- hauteurs des espaces inter-plateaux : H1 = 0,45 ; H2 = 0,30 m,
- sections des déversoirs : S1 = S2 = 1,70 m².

Pour l'exemple 4 selon l'invention, la HEPT est de 4,26 m.

La colonne d'extraction liquide-liquide présente une hauteur de 32,2 m et est composée d'une succession de 86 plateaux perforés.

### Exemple 5 (invention) : hauteurs alternées des espaces inter-plateaux et sections alternées des déversoirs

Par rapport à l'exemple 1, seules les hauteurs H1 et sections S2 sont augmentées :
- hauteurs des espaces inter-plateaux : H1 = 0,45 m ; H2 = 0,30 m,
- sections des déversoirs : S1 = 1,70 m² ; S2 = 2,10 m².

Pour l'exemple 5 selon l'invention, la HEPT est de 4,08 m.

La colonne d'extraction liquide-liquide présente une hauteur de 30,8 m et est composée d'une succession de 82 plateaux perforés.

La table 1 ci-dessous récapitule les niveaux de performance des exemples 1, 2, 3 de référence et des exemples 4 et 5 selon l'invention.

**Table 1**

| Exemple | 1 (réf.) | 2 (réf.) | 3 (réf.) | 4 (inv.) | 5 (inv.) |
|---|---|---|---|---|---|
| H1 (m) | 0,30 | 0,45 | 0,30 | 0,45 | 0,45 |
| H2 (m) | 0,30 | 0,45 | 0,30 | 0,30 | 0,30 |
| S1 (m²) | 1,70 | 1,70 | 2,10 | 1,70 | 1,70 |
| S2 (m²) | 1,70 | 1,70 | 2,10 | 1,70 | 2,10 |
| HEPT (m) | 4,76 | 5,83 | 4,81 | 4,26 | 4,08 |
| Hauteur de colonne (m) | 36,0 | 44,0 | 36,3 | 32,3 | 30,8 |
| Nombre de plateaux | 120 | 98 | 121 | 86 | 82 |

Avantageusement, la HEPT, la hauteur de colonne, et le nombre de plateaux des exemples 4 et 5 selon l'invention sont diminuées par rapport à ceux des exemples 1, 2 et 3 de référence.

## Revendications

1. Colonne d'extraction liquide-liquide comprenant les éléments suivants :
- un premier point d'injection (2) d'une première phase ;
- un deuxième point d'injection (3) d'une deuxième phase, les premier et deuxième points d'injection (2, 3) étant disposés sur la colonne d'extraction (1) pour permettre la circulation de la première phase et de deuxième phase à contre-courant dans la colonne d'extraction (1), l'une des première et deuxième phases étant une phase continue (A) et l'autre étant une phase dispersée (B) ;
- un premier point de soutirage d'un extrait (4) et un deuxième point de soutirage d'un raffinat (5), l'un étant disposé en fond de colonne d'extraction (1) et l'autre étant disposé en tête de colonne d'extraction (1) ; et
- une pluralité de plateaux perforés (Pᵢ) disposés du haut de la colonne d'extraction (1) au bas de la colonne d'extraction (1), les plateaux perforés (Pᵢ) étant espacés d'un espace inter-plateaux (8) et comprenant des rebords (10) adaptés pour recevoir une couche coalescée (9) de la phase dispersée (B) au-dessus ou au-dessous des plateaux perforés (Pᵢ) ;
- une pluralité de déversoirs (6), un déversoir (6) étant un évidement adjacent à un rebord (10) et permettant le passage la phase continue (A) au travers du plateau perforé (Pᵢ),
la colonne d'extraction (1) comprenant alternativement :
- des plateaux perforés (Pᵢ) dits de type I comprenant deux déversoirs (6) périphériques ; et
- des plateaux perforés (Pᵢ) dits de type II comprenant un seul déversoir (6) central, la colonne d'extraction (1) ;
la colonne d'extraction (1) étant **caractérisée en ce que** :
- la hauteur H1 des espaces inter-plateaux (8) disposés directement en aval des plateaux de type I, dans le sens de l'écoulement de la phase continue, est supérieure à la hauteur H2 des espaces inter-plateaux (8) disposés directement en aval des plateaux de type II.

2. Colonne d'extraction liquide-liquide selon la revendication 1, dans laquelle le ratio H1/H2 entre la hauteur H1 et la hauteur H2 est compris entre 1,1 et 2.

3. Colonne d'extraction liquide-liquide selon la revendication 1 ou la revendication 2, dans laquelle le ratio H1/H2 entre la hauteur H1 et la hauteur H2 est compris entre 1,1 et 1,5.

4. Colonne d'extraction liquide-liquide selon l'une quelconque des revendications précédentes, dans laquelle la hauteur H1 des espaces inter-plateaux (8) des plateaux perforés de type I est comprise entre 0,22 m et 1,50 m, préférablement entre 0,27 m et 0,90 m, très préférablement entre 0,33 m et 0,83 m.

5. Colonne d'extraction liquide-liquide selon l'une quelconque des revendications précédentes, dans laquelle la hauteur H2 des espaces inter-plateaux (8) des plateaux perforés de type Il est comprise entre 0,20 m et 1,00 m, préférablement entre 0,25 m et 0,60 m, très préférablement entre 0,30 m et 0,55 m

6. Colonne d'extraction liquide-liquide selon l'une quelconque des revendications précédentes, comprenant :
- un secteur d'extraction (11), s'étendant sensiblement du premier point d'injection (2) de la première phase jusqu'à sensiblement le deuxième point d'injection (3) de la deuxième phase, et
- un secteur de contre-lavage (12), adjacent au secteur d'extraction (11) et s'étendant du premier point d'injection (2) de la première phase jusqu'à sensiblement un troisième point d'injection (13) de liquide de contre-lavage.

7. Procédé d'extraction liquide-liquide comprenant les étapes suivantes :
- injecter une première phase et une deuxième phase dans la colonne d'extraction liquide-liquide selon l'une quelconque des revendications 1 à 6 ; et
- soutirer un extrait (4) et un raffinat (5) de la colonne d'extraction liquide-liquide.

8. Procédé d'extraction liquide-liquide selon la revendication 7, dans lequel la colonne d'extraction (1) est opérée à une pression comprise entre 0,05 MPa et 3 MPa, de préférence entre 0,1 MPa et 2 MPa, de préférence entre 0,2 MPa et 1,5 MPa, de préférence entre 0,3 MPa et 1 MPa ; et à une température comprise entre 10°C et 150°C, de préférence entre 15°C et 130°C, de préférence entre 30°C et 120°C, de préférence entre 40°C et 110°C.

9. Procédé d'extraction liquide-liquide selon la revendication 7 ou la revendication 8, dans lequel la première phase comprend un mélange de composés aromatiques et non-aromatiques.

10. Procédé d'extraction liquide-liquide selon l'une quelconque des revendications 7 à 9, dans lequel la deuxième phase comprend un composé choisi parmi l'éthylène glycol, le diéthylène glycol, le triéthylène glycol, hexaméthylphosphoramide, le carbonate de propylène, le carbonate d'éthylène, le sulfolane, le 3-methylsulfolane, le N-méthylacétamide, le N,N-diméthylacétamide, la butyrolactone, la 1-méthylpyrrolidone, le diméthylsulfoxyde, la caprolactame, le N-méthylformamide, la pyrrolidine-2-one, le furfural, le 1,1,3,3-tétraméthylurée et un mélange de ceux-ci.

## Patentansprüche

1. Flüssig-Flüssig-Extraktionskolonne, die die folgenden Elemente umfasst:
- einen ersten Einleitungspunkt (2) einer ersten Phase;
- einen zweiten Einleitungspunkt (3) einer zweiten Phase, wobei der erste und der zweite Einleitungspunkt (2, 3) so an der Extraktionskolonne (1) angeordnet sind, dass sie die Zirkulation der ersten Phase und der zweiten Phase im Gegenstrom in der Extraktionskolonne (1) ermöglichen, wobei eine der ersten und zweiten Phase eine kontinuierliche Phase (A) ist und die andere eine dispergierte Phase (B) ist;
- einen ersten Abzugspunkt für einen Extrakt (4) und einen zweiten Abzugspunkt für ein Raffinat (5), wobei der eine am Boden der Extraktionskolonne (1) angeordnet ist und der andere am Kopf der Extraktionskolonne (1) angeordnet ist; und
- mehrere Siebböden (Pᵢ), die vom oberen Ende der Extraktionskolonne (1) bis zum unteren Ende der Extraktionskolonne (1) angeordnet sind, wobei die Siebböden (Pᵢ) durch einen Siebzwischenraum (8) beabstandet sind und Ränder (10) aufweisen, die dazu ausgelegt sind, eine koaleszierte Schicht (9) der dispergierten Phase (B) oberhalb oder unterhalb der Siebböden (Pᵢ) aufzunehmen;
- mehrere Wehre (6), wobei ein Wehr (6) eine Aussparung neben einem Rand (10) bildet und den Durchgang der kontinuierlichen Phase (A) durch den Siebboden (Pᵢ) ermöglicht,
wobei die Extraktionskolonne (1) alternierend Folgendes umfasst:
- Siebböden (Pᵢ), die als Typ I bezeichnet werden, mit zwei peripheren Wehren (6) und
- Siebböden (Pᵢ), die als Typ II bezeichnet werden, mit einem einzigen zentralen Wehr (6),
die Extraktionskolonne (1);
wobei die Extraktionskolonne (1) **dadurch gekennzeichnet ist, dass**:
- die Höhe H1 der Siebzwischenräume (8), die unmittelbar stromabwärts der Böden des Typs I in der Strömungsrichtung der kontinuierlichen Phase angeordnet sind, größer ist als die Höhe H2 der Siebzwischenräume (8), die unmittelbar stromabwärts der Böden des Typs II angeordnet sind.

2. Flüssig-Flüssig-Extraktionskolonne nach Anspruch 1, wobei das Verhältnis H1/H2 zwischen der Höhe H1 und der Höhe H2 zwischen 1,1 und 2 liegt.

3. Flüssig-Flüssig-Extraktionskolonne nach Anspruch 1 oder Anspruch 2, wobei das Verhältnis H1/H2 zwischen der Höhe H1 und der Höhe H2 zwischen 1,1 und 1,5 liegt.

4. Flüssig-Flüssig-Extraktionskolonne nach einem der vorhergehenden Ansprüche, wobei die Höhe H1 der Siebzwischenräume (8) der Siebböden des Typs I zwischen 0,22 m und 1,50 m, vorzugsweise zwischen 0,27 m und 0,90 m, sehr bevorzugt zwischen 0,33 m und 0,83 m, liegt.

5. Flüssig-Flüssig-Extraktionskolonne nach einem der vorhergehenden Ansprüche, wobei die Höhe H2 der Siebzwischenräume (8) der Siebböden des Typs II zwischen 0,20 m und 1,00 m, vorzugsweise zwischen 0,25 m und 0,60 m, sehr bevorzugt zwischen 0,30 m und 0,55 m, liegt.

6. Flüssig-Flüssig-Extraktionskolonne nach einem der vorhergehenden Ansprüche, umfassend:
- einen Extraktionssektor (11), der sich weitgehend vom ersten Einleitungspunkt (2) der ersten Phase bis weitgehend zum zweiten Einleitungspunkt (3) der zweiten Phase erstreckt, und
- einen Rückspülsektor (12), der dem Extraktionssektor (11) benachbart ist und sich vom ersten Einleitungspunkt (2) der ersten Phase bis weitgehend zu einem dritten Einleitungspunkt (13) der Rückspülflüssigkeit erstreckt.

7. Flüssig-Flüssig-Extraktionsverfahren, das folgende Schritte umfasst:
- Einleiten einer ersten Phase und einer zweiten Phase in die Flüssig-Flüssig-Extraktionskolonne nach einem der Ansprüche 1 bis 6 und
- Abziehen eines Extrakts (4) und eines Raffinats (5) aus der Flüssig-Flüssig-Extraktionskolonne.

8. Flüssig-Flüssig-Extraktionsverfahren nach Anspruch 7, wobei die Extraktionskolonne (1) bei einem Druck zwischen 0,05 MPa und 3 MPa, vorzugsweise zwischen 0,1 MPa und 2 MPa, vorzugsweise zwischen 0,2 MPa und 1,5 MPa, vorzugsweise zwischen 0,3 MPa und 1 MPa; und bei einer Temperatur zwischen 10 °C und 150 °C, vorzugsweise zwischen 15 °C und 130 °C, vorzugsweise zwischen 30 °C und 120 °C, vorzugsweise zwischen 40 °C und 110 °C, betrieben wird.

9. Flüssig-Flüssig-Extraktionsverfahren nach Anspruch 7 oder Anspruch 8, wobei die erste Phase ein Gemisch von aromatischen und nichtaromatischen Verbindungen umfasst.

10. Flüssig-Flüssig-Extraktionsverfahren nach einem der Ansprüche 7 bis 9, wobei die zweite Phase eine Verbindung umfasst, die aus Ethylenglykol, Diethylenglykol, Triethylenglykol, Hexamethylphosphoramid, Propylencarbonat, Ethylencarbonat, Sulfolan, 3-Methylsulfolan, N-Methylacetamid, N,N-Dimethylacetamid, Butyrolacton, 1-Methylpyrrolidon, Dimethylsulfoxid, Caprolactam, N-Methylformamid, Pyrrolidin-2-on, Furfural, 1,1,3,3-Tetramethylharnstoff und einem Gemisch davon ausgewählt ist.

## Claims

1. Liquid-liquid extraction column comprising the following elements:
- a first injection point (2) for injection of a first phase;
- a second injection point (3) for injection of a second phase, the first and second injection points (2, 3) being positioned on the extraction column (1) in such a way as to allow the first and second phases to circulate in the extraction column (1) in a countercurrent manner, one of the first and second phases being a continuous phase (A) and the other being a dispersed phase (B);
- a first withdrawal point for withdrawal of an extract (4) and a second withdrawal point for withdrawal of a raffinate (5), one being located at the bottom of the extraction column (1) and the other being located at the top of the extraction column (1); and
- a plurality of sieve trays (Pᵢ) located from the top of the extraction column (1) to the bottom of the extraction column (1), the sieve trays (Pᵢ) being spaced apart by an inter-tray space (8) and comprising weir plates (10) designed to hold a layer (9) of the dispersed phase (B) that has coalesced above or below the sieve trays (Pᵢ);
- a plurality of riser/downcomer conduits (6), a riser/downcomer conduit (6) being an opening adjacent to a weir plate (10) and allowing the continuous phase (A) to pass through the sieve tray (Pᵢ),
the extraction column (1) comprising in alternation:
- sieve trays (Pᵢ) said to be of type I, comprising two peripheral riser/downcomer conduits (6); and
- sieve trays (Pᵢ) said to be of type II, comprising a single central riser/downcomer conduit (6),
the extraction column (1);
the extraction column (1) being **characterized in that**:
- the height H1 of the inter-tray spaces (8) situated directly downstream of the type-I trays, in the direction of flow of the continuous phase, is greater than the height H2 of the inter-tray spaces (8) positioned directly downstream of the type-II trays.

2. Liquid-liquid extraction column according to Claim 1, wherein the ratio H1/H2 of the height H1 to the height H2 is comprised between 1.1 and 2.

3. Liquid-liquid extraction column according to Claim 1 or Claim 2, wherein the ratio H1/H2 of the height H1 to the height H2 is comprised between 1.1 and 1.5.

4. Liquid-liquid extraction column according to any one of the preceding claims, wherein the height H1 of the inter-tray spaces (8) of the type-I sieve trays is comprised between 0.22 m and 1.50 m, preferably between 0.27 m and 0.90 m, very preferably between 0.33 m and 0.83 m.

5. Liquid-liquid extraction column according to any one of the preceding claims, wherein the height H2 of the inter-tray spaces (8) of the type-II sieve trays is comprised between 0.20 m and 1.00 m, preferably between 0.25 m and 0.60 m, very preferably between 0.30 m and 0.55 m.

6. Liquid-liquid extraction column according to any one of the preceding claims, comprising:
- an extraction sector (11), extending substantially from the first injection point (2) for injection of the first phase as far as substantially the second injection point (3) for injection of the second phase, and
- a backwash sector (12), adjacent to the extraction sector (11) and extending from the first injection point (2) for injection of the first phase as far as substantially a third injection point (13) for injection of a backwash liquid.

7. Liquid-liquid extraction method comprising the following steps:
- injecting a first phase and a second phase into the liquid-liquid extraction column according to any one of Claims 1 to 6; and
- withdrawing an extract (4) and a raffinate (5) from the liquid-liquid extraction column.

8. Liquid-liquid extraction method according to Claim 7, wherein the extraction column (1) is operated at a pressure comprised between 0.05 MPa and 3 MPa, preferably between 0.1 MPa and 2 MPa, preferably between 0.2 MPa and 1.5 MPa, and preferably between 0.3 MPa and 1 MPa; and at a temperature comprised between 10°C and 150°C, preferably between 15°C and 130°C, preferably between 30°C and 120°C, and preferably between 40°C and 110°C.

9. Liquid-liquid extraction method according to Claim 7 or Claim 8, wherein the first phase comprises a mixture of aromatic and nonaromatic compounds.

10. Liquid-liquid extraction method according to any one of Claims 7 to 9, wherein the second phase contains a compound selected from ethylene glycol, diethylene glycol, triethylene glycol, hexamethylphosphoramide, propylene carbonate, ethylene carbonate, sulfolane, 3-methylsulfolane, N-methylacetamide, N,N-dimethylacetamide, butyrolactone, 1-methylpyrrolidone, dimethyl sulfoxide, caprolactam, N-methylformamide, pyrrolidin-2-one, furfural, 1,1,3,3-tetramethylurea and a mixture of these.
